(19) Europäisches Patentamt
European Patent Office
Office européen des brevets

(11) **EP 2 796 094 B1**

(12) **FASCICULE DE BREVET EUROPEEN**

(45) Date de publication et mention
de la délivrance du brevet:
**20.10.2021 Bulletin 2021/42**

(51) Int Cl.:
***A61B 6/00*** (2006.01)

(21) Numéro de dépôt: **14165323.8**

(22) Date de dépôt: **18.04.2014**

(54) **Procédé et dispositif de correction/calibration d'un paramètre de caractérisation d'un tissu osseux**

Verhahren und Vorrichtung zur Korrektur/Kalibrierung eines Charakterisierungsparameters eines Knochengewebes

Method and apparatus for correcting/calibrating a parameter for characterising bone tissue

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.04.2013 FR 1353696**

(43) Date de publication de la demande:
**29.10.2014 Bulletin 2014/44**

(73) Titulaire: **Medimaps Group SA**
**1228 Plan-Les-Ouates, Geneve (CH)**

(72) Inventeur: **Chaintreuil, Jean**
**Orsay (FR)**

(74) Mandataire: **IPAZ**
**Parc Les Algorithmes, Bâtiment Platon**
**CS 70003 Saint-Aubin**
**91192 Gif-sur-Yvette Cedex (FR)**

(56) Documents cités:
**US-A1- 2007 047 794    US-B1- 6 377 653**

• **CALIGIURI P ET AL: "MULTIFRACTAL RADIOGRAPHIC ANALYSIS OF OSTEOPOROSIS", MEDICAL PHYSICS, AIP, MELVILLE, NY, US, vol. 21, no. 4, 1 avril 1994 (1994-04-01), pages 503-508, XP000450819, ISSN: 0094-2405, DOI: 10.1118/1.597390**
• **DOYLE M ET AL: "THE USE OF FRACTAL ANALYSIS IN THE SCREENING OF MEDICAL/DENTAL X- RAY AND TOMOGRAPHIC IMAGES FOR EARLY SIGNS OF OSTEOPOROSIS", PROCEEDINGS OF THE ANNUAL INTERNATIONAL CONFERENCE OF THE ENGINEERING IN MEDICINE AND BIOLOGY SOCIETY. ORLANDO, OCT. 31 - NOV. 3, 1991; [PROCEEDINGS OF THE ANNUAL INTERNATIONAL CONFERENCE OF THE ENGINEERING IN MEDICINE AND BIOLOGY SOCIETY], NEW YORK,, vol. 13, no. PART 05/05, 31 octobre 1991 (1991-10-31), XP000347770,**
• **LESPESSAILLES E ET AL: "Clinical interest of bone texture analysis in osteoporosis: a case control multicenter study", OSTEOPOROSIS INTERNATIONAL ; WITH OTHER METABOLIC BONE DISEASES, SPRINGER-VERLAG, LO, vol. 19, no. 7, 15 January 2008 (2008-01-15), pages 1019-1028, XP019620429, ISSN: 1433-2965**

EP 2 796 094 B1

## Description

### Domaine technique

La présente invention concerne un procédé de correction/calibration d'un paramètre de caractérisation d'un tissu osseux. Elle concerne également un système mettant en œuvre un tel procédé.

Le domaine de l'invention est le domaine médical et plus précisément le domaine de l'étude des tissus osseux avec des appareils d'imagerie comprenant un émetteur de rayons de particules électriquement neutres et mettant en œuvre un capteur de rayons. L'invention s'applique plus particulièrement au domaine de l'imagerie médicale de haute précision et de l'étude de la caractérisation des os.

### Etat de ma technique

Actuellement, il existe de nombreux appareils d'imagerie de tissus osseux. Ces appareils comprennent une source de particules électriquement neutres prévue pour émettre un rayonnement et éventuellement un récepteur doté d'un capteur numérique ou analogique. Le tissu osseux est positionné entre l'émetteur et le récepteur.

Le procédé mis en œuvre dans ces appareils comprend une émission d'un rayonnement de particules électriquement neutres qui viennent frapper le tissu osseux. Les particules électriquement neutres traversent plus ou moins le milieu étudié en fonction de sa densité. L'atténuation est plus importante dans un milieu osseux que dans les tissus mous.

L'image obtenue par ces procédés est une image en niveaux de gris et le niveau de gris en un point de l'image obtenue dépend de la quantité de particules frappant le capteur en ce point. L'image en niveaux de gris est ensuite analysée et traitée pour déterminer la valeur d'un ou plusieurs paramètres relatifs au milieu osseux, aussi appelé « paramètre de caractérisation » dans la présente demande. Un tel paramètre de caractérisation peut par exemple être le paramètre de texture H, aussi connu sous le nom de paramètre de Hurst. Un exemple de procédé est décrit dans le document CALIGIURI P ET AL: "Multifractal Radiographie Analysis Of Osteoporosis",MEDICAL PHYSICS, AIP, MELVILLE, NY, US, vol. 21, no. 4, 1 avril 1994 (1994-04-01), pages 503-508,ISSN: 0094-2405, DOI: 10.1118/1.597390.

La valeur mesurée d'un paramètre de caractérisation d'un tissu osseux dépend fortement des conditions de mesure, et plus particulièrement du capteur utilisé pour générer l'image en niveaux de gris et de la dose de particules émise pour l'acquisition de l'image en niveaux de gris. Par conséquent il est difficile, voire impossible, de comparer, pour un même paramètre de caractérisation, deux valeurs mesurées dans deux configurations de mesures différentes.

Actuellement, pour réaliser une telle comparaison, l'une des valeurs mesurées est calibrée/corrigée en utilisant une régression linéaire de type Mesure (configuration 1)= constante* Mesure (Configuration 2) s'appliquant directement aux valeurs mesurées du paramètre de caractérisation. Cependant, une telle calibration/correction donne des valeurs très approximatives, voire aberrantes, et ne peut donc être utilisée.

Le document US 6 377 653 décrit un procédé de calibrage d'un indice trabéculaire visant à tenir compte des conditions d'acquisition de l'image à partir de laquelle est déterminé cet indice trabéculaire.

Un but de l'invention est de pallier les inconvénients cités ci-dessus.

Un autre but de l'invention est de proposer un procédé et système de calibration/correction réalisant une calibration/correction de la valeur mesurée d'un paramètre de caractérisation d'un tissu osseux en fonction de la configuration de mesure utilisée, fournissant de meilleurs résultats quelle que soit la configuration de mesure.

Un autre but de la présente invention est de proposer un procédé et système de calibration/correction réalisant une calibration/correction de la valeur mesurée d'un paramètre de caractérisation d'un tissu osseux en fonction de la configuration de mesure utilisée, fournissant de meilleurs résultats quel(le) que soit le capteur utilisé ou la dose de particules utilisées.

Enfin, un autre but de l'invention est de proposer un procédé et système de mesure d'un paramètre de caractérisation d'un tissu osseux mettant en œuvre une telle calibration/correction.

### Exposé de l'invention

L'invention propose d'atteindre au moins l'un de ces buts par un procédé tel que défini dans la revendication indépendante 1.

Le procédé selon l'invention propose de réaliser une correction d'une valeur mesurée d'un paramètre de caractérisation d'un tissu osseux à partir de la dimension fractale de l'image en niveau de gris utilisée pour déterminer ladite valeur mesurée. Le procédé selon l'invention permet donc de réaliser la correction/calibration du paramètre de caractérisation de manière indirecte, c'est-à-dire sans utiliser une relation qui s'applique directement sur la valeur mesurée du paramètre de caractérisation, alors que les procédés de l'état de la technique réalisent une correction se basant directement sur la valeur mesurée du paramètre de caractérisation.

L'inventeur de la présente invention a découvert que la dimension fractale de l'image en niveaux de gris constitue une base permettant de réaliser une meilleure correction/calibration prenant mieux en compte les différences dans les configurations de mesure comparée à une correction/calibration basée sur la valeur mesurée du paramètre de caractérisation.

La correction/calibration selon l'invention permet de fournir des résultats répétables et confirmés par des essais cliniques, permettant ainsi une comparaison correcte de valeurs mesurées dans différentes configurations de mesure.

Selon tous les aspects de l'invention, la dimension fractale d'une image en niveaux de gris peut être déterminée :

- par analyse de l'image en niveaux de gris, et/ou
- suivant une relation prédéterminée prenant en compte la valeur mesurée du paramètre de caractérisation.

Selon l'invention, l'étape de correction de la dimension fractale mesurée, par rapport à la configuration de référence, peut être réalisée suivant une relation prédéterminée de manière empirique avec plusieurs échantillons de mesure pour lesquels les valeurs du paramètre de caractérisation, et/ou les valeurs de la dimension fractale, sont connues dans la configuration de mesure et dans la configuration de référence.

Une telle relation prédéterminée peut être une régression linéaire prenant en compte la dimension fractale de l'image en niveaux de gris obtenue dans la configuration de mesure et la dimension fractale de l'image en niveaux de gris obtenue dans la configuration de référence.

Un exemple de relation prédéterminée peut être la suivante :

$$D_m = A\ D_r$$

avec $D_m$ la dimension fractale de l'image en niveaux de gris obtenue dans la configuration de mesure, $D_{ref}$ la dimension fractale de l'image en niveaux de gris obtenue dans la configuration de référence, et A une constante déterminée de manière empirique, et qui correspond à la pente de la régression linéaire passant par l'origine réalisée entre Dm et Dr.

Selon un premier exemple de réalisation, la configuration de mesure peut différer de la configuration de référence par le détecteur ou capteur utilisé pour réaliser l'image en niveau de gris.

Chaque détecteur/capteur peut être numérique ou analogique.

Dans le cas où le détecteur est un détecteur analogique, le procédé selon l'invention peut en outre comprendre une numérisation de l'image en niveaux de gris.

Selon un autre exemple de réalisation, la configuration de mesure peut différer de la configuration de référence par la quantité et/ l'énergie des particules utilisées pour réaliser l'image en niveau de gris.

Bien entendu, la configuration de mesure peut différer de la configuration de référence par au moins deux paramètres à la fois, par exemple à la fois par le détecteur utilisé et par la dose de particules utilisées. Dans ce cas, la correction/calibration réalisée prend en compte la combinaison de chacune de ces différences.

Selon un autre aspect de l'invention, il est proposé un procédé de détermination/mesure d'un paramètre de caractérisation d'un tissu osseux, ledit procédé comprenant les étapes suivantes :

- acquisition d'une image en niveaux de gris dudit tissu osseux, et

- détermination d'une valeur, dite corrigée/calibrée, dudit paramètre de caractérisation suivant par le procédé selon l'invention.

La valeur mesurée du paramètre de caractérisation peut en particulier être réalisée dans une région, dite d'intérêt, dudit tissu osseux préalablement identifiée par l'intermédiaire de repères anatomique.

Une telle région d'intérêt peut être définie et identifiée sur une image en niveaux de gris préalablement acquise. Dans ce cas, le procédé de détermination/mesure selon l'invention comprend une étape préalable d'acquisition d'une première image en niveaux de gris, puis une identification de la région d'intérêt par des marqueurs anatomiques puis une acquisition d'une deuxième image en niveaux de gris, centrée sur la région d'intérêt. La détermination de la valeur mesurée du paramètre de caractérisation est alors réalisée sur cette deuxième image en niveaux de gris centrée sur la région d'intérêt.

Selon l'invention, le paramètre de caractérisation du tissu osseux déterminé est un paramètre de texture osseuse, tel que le paramètre de Hurst. La présente invention ne concerne pas la détermination d'autres types de paramètre de caractérisation tels que la densité osseuse, ou tout paramètre autre que la texture osseuse et dont la valeur est déterminée par mesure fractale sur une image en niveaux de gris.

Selon encore un autre aspect de l'invention, il est proposé un dispositif tel que défini dans la revendication indépendante 7.

Selon encore un autre aspect de l'invention il est proposé un système de détermination/mesure d'un paramètre de caractérisation d'un tissu osseux, ledit système comprenant :

- des moyens d'imagerie pour l'acquisition d'au moins une image en niveaux de gris dudit tissu osseux, et
- un dispositif de correction/calibration selon l'invention.

Les moyens d'imagerie peuvent comprendre au moins un détecteur qui peut être un détecteur numérique ou analogique.

Dans le cas où, le détecteur est un détecteur analogique, le système selon l'invention peut en outre comprendre des moyens de numérisation de l'image analogique fournie par le détecteur analogique.

Le dispositif, respectivement le système, selon l'invention peut être mis en oeuvre, pour la correction/calibration, respectivement la détermination/mesure, de la texture osseuse, définie par un paramètre dérivé de la mesure d'une dimension fractale de l'image, par exemple, le paramètre de Hurst.

## Description des figures et modes de réalisation

D'autres avantages et caractéristiques apparaîtront à l'examen de la description détaillée d'un mode de réalisation nullement limitatif, et des dessins annexés sur lesquels

- la figure 1 est une représentation sous la forme d'un diagramme des étapes d'un exemple de procédé de correction/calibration selon l'invention de la valeur mesurée d'un paramètre de caractérisation d'un tissu osseux ;
- la figure 2 est une représentation sous la forme d'un diagramme des étapes d'un exemple de procédé de détermination/mesure selon l'invention de la valeur d'un paramètre de caractérisation d'un tissu osseux ;
- la figure 3 est une représentation schématique d'un dispositif selon l'invention ;
- la figure 4 est une représentation schématique d'un système selon l'invention, et
- les figures 5 et 6 correspondent chacune à une représentation schématique d'un exemple de résultats obtenus selon l'invention.

Il est bien entendu que les modes de réalisation qui seront décrits dans la suite ne sont nullement limitatifs.

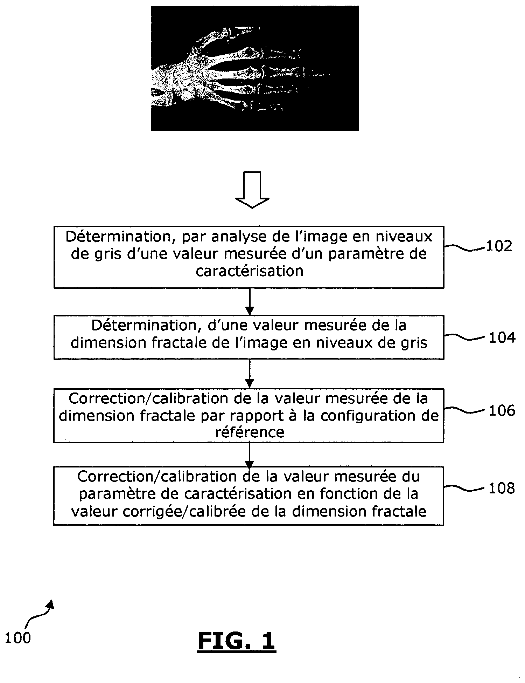

La FIGURE 1 est une représentation sous la forme d'un diagramme des étapes d'un exemple non limitatif d'un procédé de correction/calibration selon l'invention de la valeur mesurée d'un paramètre de caractérisation d'un tissu osseux.

Dans l'exemple donné en FIGURE 1, la configuration de mesure diffère d'une configuration de référence prédéterminée soit par le capteur/détecteur utilisé, soit par la dose utilisée en émission pour l'acquisition de l'image en niveau de gris, ou encore les deux.

Le procédé 100 représenté sur la FIGURE 1 comprend une étape 102 réalisant une mesure d'une valeur, dite mesurée, d'un paramètre de caractérisation, par analyse d'une image en niveaux de gris d'un tissu osseux. Dans l'exemple présent le paramètre de caractérisation est, à titre d'exemple uniquement et de manière nullement limitative, le paramètre de Hurst H et la valeur mesurée est notée $H_1$.

Lors d'une étape 104, une valeur $D_1$, dite mesurée, de la dimension fractale D de l'image en niveaux de gris est déterminée. La dimension fractale D est déterminée soit par analyse de l'image en niveaux de gris, soit à partir de la valeur mesurée $H_1$ du paramètre de Hurst, selon une relation prédéterminée. Dans l'exemple

présent, la valeur $D_1$ est déterminée à partir de la valeur $H_1$ selon la relation prédéterminée :

$$D_1 = 2 - H_1 \quad \textbf{(1)}$$

Lors d'une étape 106, la dimension fractale mesurée $D_1$ est corrigée par rapport à une configuration de référence.

La valeur mesurée $D_1$ est corrigée suivant une relation linéaire préalablement déterminée de manière empirique et reliant la valeur de la dimension fractale dans la configuration de référence et la configuration de mesure, et en particulier en fonction de la pente de la régression linéaire passant par l'origine réalisée entre les valeurs de la dimension fractale dans la configuration de référence et dans la configuration de mesure. Cette relation linéaire est définie en mesurant, pour des échantillons connus, la valeur de la dimension fractale dans la configuration de référence et la configuration de mesure.

Dans l'exemple présent, la valeur corrigée $D_{cor}$ de la dimension fractale mesurée est obtenue suivant la relation :

$$D_{cor} = D_1 * 1,17 \quad \textbf{(2)}$$

Lors d'une étape 108 la valeur corrigée/calibrée du paramètre de caractérisation $H_{cor}$ est déterminée à partie de la valeur corrigée/calibrée $D_{cor}$ de la dimension fractale, par exemple en utilisant la relation (1) ci-dessus, à savoir :

$$H_{cor} = 2 - D_{cor} \quad \textbf{(3)}$$

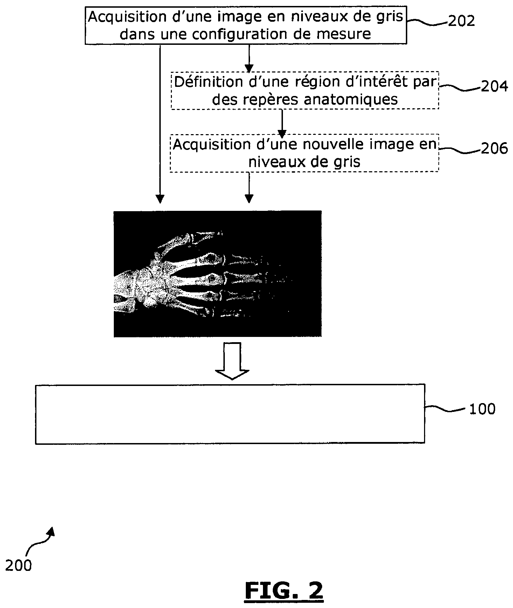

La FIGURE 2 est une représentation sous la forme d'un diagramme des étapes d'un exemple non limitatif d'un procédé de détermination/mesure de la valeur d'un paramètre de caractérisation d'un tissu osseux selon l'invention.

Le procédé 200 représenté sur la FIGURE 2 comprend une étape 202 d'acquisition d'une image en niveaux de gris d'un tissu osseux.

Cette étape 202, peut être suivie d'une étape optionnelle 204 lors de laquelle une région d'intérêt est définie sur l'image en niveau de gris acquise lors de l'étape 202, par des repères anatomiques.

Cette étape optionnelle 204 est alors suivie d'une étape 206 d'acquisition d'une nouvelle image en niveaux de gris centrée autour/sur la région d'intérêt. Cette étape 206 n'est réalisée que si l'étape optionnelle 204 de définition d'une région d'intérêt est réalisée.

L'étape 202, le cas échéant l'étape 206, est suivie des étapes du procédé 100 représenté en figure 1, déterminant une valeur corrigée/calibrée d'une valeur d'un paramètre de caractérisation d'un tissu osseux.

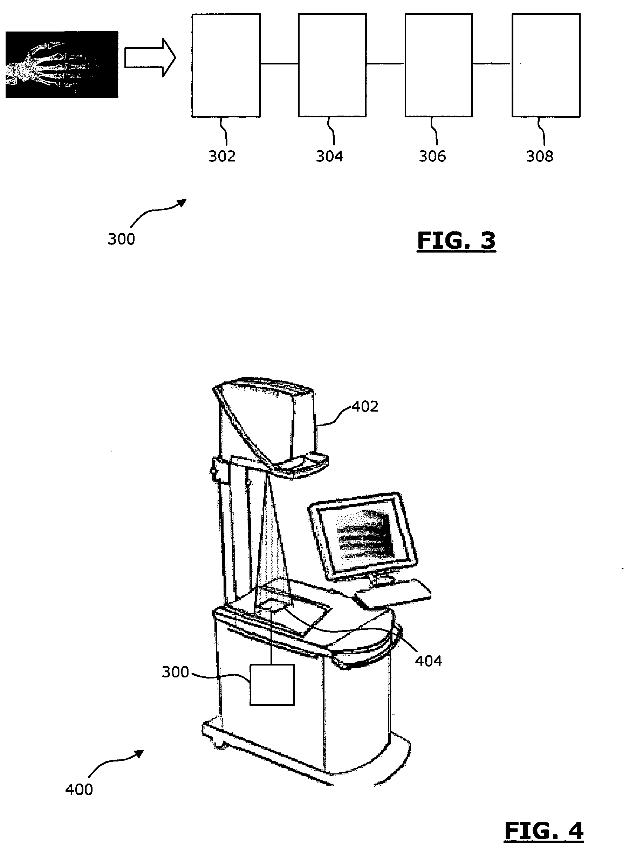

La FIGURE 3 est une représentation schématique d'un exemple de dispositif selon l'invention.

Le dispositif 300, représenté sur le FIGURE 3, comprend :

- un module 302 de mesure d'une valeur, dite mesurée, d'un paramètre de caractérisation, à partir d'une image en niveaux de gris,
- un module 304 de mesure d'une valeur, dite mesurée, de la dimension fractale de l'image en niveau de gris à partir soit de l'image en niveaux de gris soit à partir de la valeur mesurée du paramètre de caractérisation,
- un module 306 de détermination par calcul d'une valeur corrigée de la dimension fractale par rapport à une configuration de référence, par exemple suivant une relation prédéterminée de manière empirique prenant en compte la valeur mesurée de la dimension fractale, et
- un module 308 de détermination par calcul d'une valeur corrigée du paramètre de caractérisation prenant en compte la valeur corrigée de la dimension fractale.

Chaque module peut être un moyen de calcul, tel qu'un processeur, agencé pour exécuter un programme informatique ou des lignes de commandes dédiées à l'étape ou l'opération à réaliser.

Bien que représentés séparément, au moins deux des modules, en particulier tous les modules, peuvent être intégrés dans un même processeur.

La FIGURE 4 est une représentation schématique d'un exemple de système selon l'invention.

Le système 400 représenté sur la FIGURE 4 comprend un émetteur 402 pour émettre une dose de particules neutres et un récepteur 404, placé en regard de l'émetteur 402, pour l'acquisition d'une image en niveaux de gris. Le tissu osseux à caractériser est disposé entre l'émetteur 402 et le récepteur 404, de sorte que les particules émises par l'émetteur 402 traversent le tissu osseux avant d'arriver sur le récepteur 404.

Le système 400 comprend en outre un dispositif 300 tel que représenté sur la FIGURE 3 relié au récepteur 404.

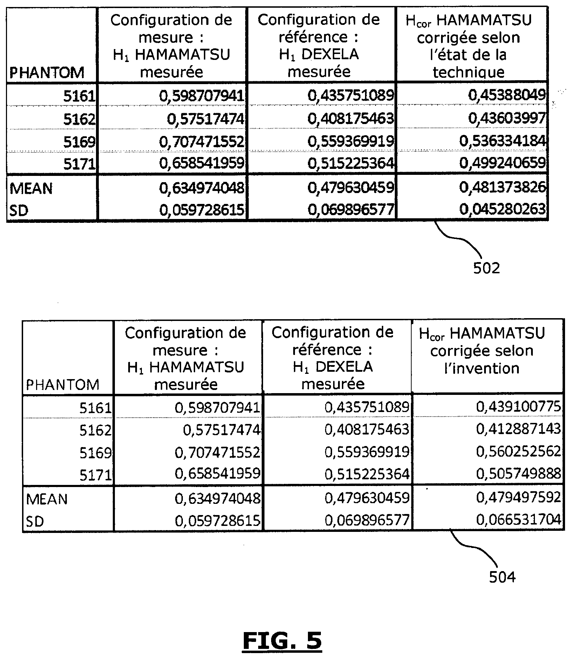

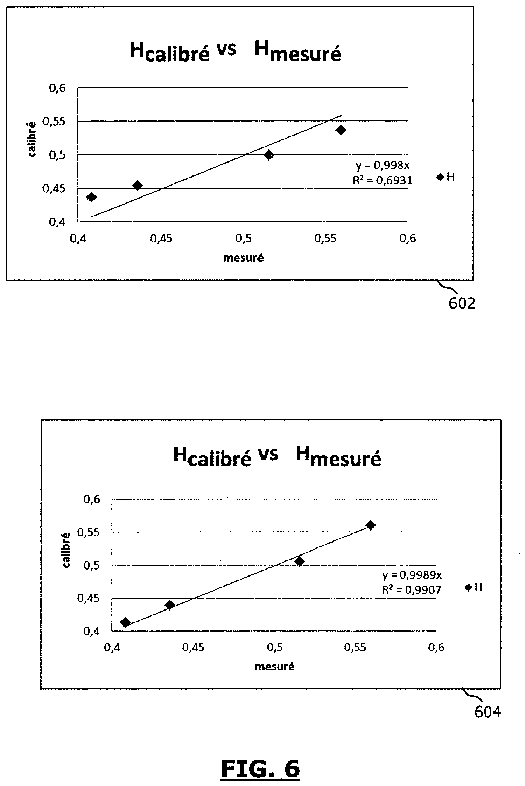

Les FIGURES 5 et 6 sont chacune une représentation schématique d'un exemple de résultats obtenus selon l'invention.

Les résultats représentés sur les FIGURES 5 et 6 correspondent à la mesure du paramètre de texture H, pour plusieurs échantillons connus appelés PHANTOM, réalisée dans une configuration de mesure qui diffère d'une configuration de référence uniquement par le capteur utilisé.

Dans la configuration de mesure le capteur utilisé est un capteur de marque HAMAMTSU et dans la configuration de référence le capteur utilisé est un capteur de marque DEXELA.

Le tableau 502 donne, pour chaque PHANTOM, la valeur corrigée/calibrée du paramètre H selon l'invention

les procédés de l'état de la technique basée directement sur la valeur mesurée. Le tableau 504 donne, pour chaque PHANTOM, la valeur corrigée/calibrée du paramètre H selon l'invention.

Le graphique 602 montre la valeur calibrée du paramètre de texture H obtenue avec les techniques de calibration de l'état de l'art en fonction de la valeur mesurée. Le graphique 604 montre la valeur calibrée du paramètre de texture H obtenue avec l'invention en fonction de la valeur mesurée.

Les tableaux 502 et 504 montrent que la correction/calibration selon l'invention est meilleure que la correction/calibration selon les procédés de l'état de la technique, et fournit des valeurs corrigées plus proches de la configuration de référence.

Ceci est également illustré par la figure 6.

De plus, en ce qui concerne la valeur SD de déviation standard des niveaux de gris et la valeur MEAN de niveau de gris moyen, les valeurs corrigées obtenues par la correction/calibration selon l'invention sont en adéquation avec les valeurs de SD et de MEAN obtenues dans la configuration de référence. Pour les procédés de l'état de la technique, les valeurs corrigées de SD et de MEAN sont très différentes des valeurs obtenues dans la configuration de référence.

Dans les exemples donnés le paramètre de caractérisation considéré est le paramètre de texture H. Bien entendu, l'invention n'est pas limitée au paramètre de texture H et peut être appliquée à tout paramètre de texture osseuse mesuré par analyse fractale.

L'invention n'est bien sûr pas limitée aux exemples qui viennent d'être décrits, mais définie par les revendications.

**Revendications**

1. Procédé (100) de correction/calibration, mis en œuvre par au moins un moyen de calcul, d'une valeur, dite mesurée, d'un paramètre de caractérisation d'un tissu osseux déterminée par mesure fractale sur une image en niveaux de gris réalisée dans une première configuration, dite de mesure, par rapport à une deuxième configuration, dite de référence, se distinguant de ladite configuration de mesure par une différence affectant la valeur mesurée dudit paramètre de caractérisation, ledit procédé étant **caractérisé en ce que** le paramètre de caractérisation est un paramètre de texture osseuse, et **en ce qu'**il comprend les étapes suivantes :

   - détermination (104) d'une dimension fractale, dite mesurée, de ladite image en niveaux de gris réalisée dans la configuration de mesure,
   - correction (106) de ladite dimension fractale mesurée en référence à ladite configuration de référence, ladite correction fournissant une valeur dite corrigée de ladite dimension fractale,

- détermination (108) d'une valeur, dite corrigée, dudit paramètre de caractérisation à partir de ladite valeur corrigée de ladite dimension fractale.

2. Procédé (100) selon la revendication 1, **caractérisé en ce que** l'étape de correction de la dimension fractale mesurée, par rapport à la configuration de référence, est réalisée suivant une relation prédéterminée de manière empirique avec plusieurs échantillons de mesure pour lesquels les valeurs du paramètre de caractérisation et/ou les valeurs de la dimension fractale sont connues dans la configuration de mesure et dans la configuration de référence.

3. Procédé (100) selon l'une quelconque des revendications précédentes, **caractérisé en ce que** la configuration de mesure diffère de la configuration de référence par le détecteur utilisé pour réaliser l'image en niveau de gris.

4. Procédé (100) selon l'une quelconque des revendications précédentes, **caractérisé en ce que** la configuration de mesure diffère de la configuration de référence par la quantité et/ou l'énergie des particules utilisées pour réaliser l'image en niveau de gris.

5. Procédé (200) de détermination d'un paramètre de caractérisation d'un tissu osseux, ledit procédé (200) comprenant les étapes suivantes :

   - acquisition (202-206) d'une image en niveaux de gris dudit tissu osseux, et
   - détermination (100) d'une valeur, dite corrigée/calibrée, dudit paramètre de caractérisation suivant le procédé (100) selon l'une quelconque des revendications précédentes.

6. Procédé (200) selon l'une quelconque des revendications précédentes, **caractérisé en ce que** le paramètre de texture osseuse est le paramètre de Hurst.

7. Dispositif (300) de correction/calibration d'une valeur, dite mesurée, d'un paramètre de caractérisation d'un tissu osseux déterminée par mesure fractale sur une image en niveaux de gris réalisée dans une première configuration, dite de mesure, par rapport à une deuxième configuration, dite de référence, se distinguant de ladite configuration de mesure par une différence affectant la valeur mesurée dudit paramètre de caractérisation, ledit dispositif (300) étant **caractérisé en ce que** le paramètre de caractérisation est un paramètre de texture osseuse, et **en ce qu'**il comprend des moyens (302-308) comprenant au moins un moyen de calcul et agencés et configurés pour :

   - déterminer une dimension fractale de ladite image en niveaux de gris,
   - corriger ladite dimension fractale en référence à ladite configuration de référence, ladite correction fournissant une valeur dite corrigée de ladite dimension fractale, et
   - déterminer une valeur, dite corrigée, dudit paramètre de caractérisation à partir de ladite valeur corrigée de ladite dimension fractale.

8. Dispositif (300) de correction/calibration selon la revendication précédente, dans lequel le paramètre de texture osseuse est le paramètre de Hurst.

9. Système (400) de détermination d'un paramètre de caractérisation d'un tissu osseux, ledit système (400) comprenant :

   - des moyens (402, 404) d'imagerie pour l'acquisition d'une image en niveaux de gris dudit tissu osseux, et
   - un dispositif (300) de correction/calibration selon l'une des revendications 7 et 8.

10. Système (400) selon la revendication précédente, **caractérisé en ce que** les moyens d'imagerie (402, 404) comprennent un détecteur (404) numérique ou analogique.

11. Utilisation du système selon l'une quelconque des revendications 9 et 10 pour la détermination de la texture osseuse d'un tissu osseux.


## Patentansprüche

1. Verfahren (100), durchgeführt mittels mindestens eines Rechenmittels, zur Korrektur/Kalibrierung eines als Messwert bezeichneten Wertes eines Charakterisierungsparameters eines Knochengewebes, der durch Fraktalmessung an einem Graustufenbild bestimmt wird, das in einer ersten Konfiguration, Messkonfiguration genannt, in Bezug auf eine zweite Konfiguration, Referenzkonfiguration genannt, erzeugt wird, die sich von der Messkonfiguration durch einen Unterschied unterscheidet, der den Messwert des Charakterisierungsparameters beeinflusst, wobei das Verfahren **dadurch gekennzeichnet ist, dass** der Charakterisierungsparameter ein Parameter der Knochenbeschaffenheit ist, und dadurch, dass es die folgenden Schritte umfasst:

   - Bestimmung (104) einer als gemessen bezeichneten fraktalen Dimension des in der Messkonfiguration erzeugten Graustufenbildes,
   - Korrektur (106) der gemessenen fraktalen Dimension in Bezug auf die Referenzkonfiguration, wobei die Korrektur einen als korrigiert bezeichneten Wert der fraktalen Dimension liefert,
- Bestimmen (108) eines als korrigiert bezeichneten Wertes des Charakterisierungsparameters basierend auf dem korrigierten Wert der fraktalen Dimension.

2. Verfahren (100) nach Anspruch 1, **dadurch gekennzeichnet, dass** der Schritt der Korrektur der gemessenen fraktalen Dimension in Bezug auf die Referenzkonfiguration gemäß einer mit mehreren Messproben empirisch vorbestimmten Beziehung durchgeführt wird, für die die Werte des Charakterisierungsparameters und/oder die Werte der fraktalen Dimension in der Messkonfiguration und in der Referenzkonfiguration bekannt sind.

3. Verfahren (100) nach einem der vorhergehenden Ansprüche, **dadurch gekennzeichnet, dass** sich die Messkonfiguration von der Referenzkonfiguration durch den Detektor unterscheidet, der zur Erzeugung des Graustufenbildes verwendet wird.

4. Verfahren (100) nach einem der vorhergehenden Ansprüche, **dadurch gekennzeichnet, dass** sich die Messkonfiguration von der Referenzkonfiguration durch die Quantität und/oder die Energie von Teilchen unterscheidet, die zur Erzeugung des Graustufenbildes verwendet werden.

5. Verfahren (200) zur Bestimmung eines Charakterisierungsparameters eines Knochengewebes, wobei das Verfahren (200) die folgenden Schritte umfasst:

   - Aufnahme (202-206) eines Graustufenbildes des Knochengewebes, und
   - Bestimmung (100) eines als korrigiert/kalibriert bezeichneten Wertes des Charakterisierungsparameters gemäß des Verfahrens (100) nach einem der vorhergehenden Ansprüche.

6. Verfahren (200) nach einem der vorhergehenden Ansprüche, **dadurch gekennzeichnet, dass** der Parameter der Knochenbeschaffenheit der Hurst-Parameter ist.

7. Vorrichtung (300) zur Korrektur/Kalibrierung eines als Messwert bezeichneten Wertes eines Charakterisierungsparameters eines Knochengewebes, der durch Fraktalmessung an einem Graustufenbild bestimmt wird, das in einer ersten Konfiguration, Messkonfiguration genannt, in Bezug auf eine zweite Konfiguration, Referenzkonfiguration genannt, erzeugt wird, die sich von der Messkonfiguration durch einen Unterschied unterscheidet, der den Messwert des Charakterisierungsparameters beeinflusst, wobei die Vorrichtung (300) **dadurch gekennzeichnet ist, dass** der Charakterisierungsparameter ein Parameter der Knochenbeschaffenheit ist, und dadurch, dass diese Mittel (302-308) umfasst, die mindestens ein Rechenmittel umfassen und ausgelegt und konfiguriert sind zum:

   - Bestimmen einer fraktalen Dimension des Graustufenbildes,
   - Korrigieren der fraktalen Dimension in Bezug auf die Referenzkonfiguration, wobei die Korrektur einen als korrigiert bezeichneten Wert der fraktalen Dimension liefert, und
   - Bestimmen eines als korrigiert bezeichneten Wertes des Charakterisierungsparameters basierend auf dem korrigierten Wert der fraktalen Dimension.

8. Vorrichtung (300) zur Korrektur/Kalibrierung nach dem vorhergehenden Anspruch, wobei der Parameter der Knochenbeschaffenheit der Hurst-Parameter ist.

9. System (400) zur Bestimmung eines Charakterisierungsparameters von Knochengewebe, wobei das System (400) umfasst:

   - Mittel (402, 404) zur Bildgebung zur Aufnahme eines Graustufenbildes des Knochengewebes, und
   - eine Vorrichtung (300) zur Korrektur/Kalibrierung nach einem der Ansprüche 7 und 8.

10. System (400) nach dem vorhergehenden Anspruch, **dadurch gekennzeichnet, dass** die Bildgebungsmittel (402, 404) einen digitalen oder analogen Detektor (404) umfassen.

11. Verwendung des Systems nach einem der Ansprüche 9 und 10 zur Bestimmung der Knochenbeschaffenheit eines Knochengewebes.

## Claims

1. Method (100) for correcting/calibrating, implemented by at least one calculation means, a value, called measured value, of a parameter for characterizing bone tissue determined by fractal measurement on a greyscale image produced in a first configuration, called measurement configuration, with respect to a second configuration, called reference configuration, distinguished from said measurement configuration by a difference affecting the measured value of said characterizing parameter, said method being **characterized in that** the characterizing parameter is a bone texture parameter, and **in that** it comprises the following steps:

   - determining (104) a fractal dimension, called measured fractal dimension, of said greyscale image produced in the measurement configuration,
- correcting (106) said measured fractal dimension with reference to said reference configuration, said correction providing a value, called corrected value, of said fractal dimension,
- determining (108) a value, called corrected value, of said characterizing parameter from said corrected value of said fractal dimension.

2. Method (100) according to claim 1, **characterized in that** the step of correcting the measured fractal dimension, with respect to the reference configuration, is carried out according to a relationship predetermined empirically with several measurement samples for which the values of the characterizing parameter and/or the values of the fractal dimension are known in the measurement configuration and in the reference configuration.

3. Method (100) according to any one of the preceding claims, **characterized in that** the measurement configuration differs from the reference configuration by the detector used to produce the greyscale image.

4. Method (100) according to any one of the preceding claims, **characterized in that** the measurement configuration differs from the reference configuration by the quantity and/or the energy of the particles used to produce the greyscale image.

5. Method (200) for determining a parameter characterizing bone tissue, said method (200) comprising the following steps:

- acquiring (202-206) a greyscale image of said bone tissue, and
- determining (100) a value, called corrected/calibrated value, of said characterizing parameter following the method (100) according to any one of the preceding claims.

6. Method (200) according to any one of the preceding claims, **characterized in that** the bone texture parameter is the Hurst parameter.

7. Apparatus (300) for correcting/calibrating a value, called measured value, of a parameter for characterizing bone tissue determined by fractal measurement on a greyscale image produced in a first configuration, called measurement configuration, with respect to a second configuration, called reference configuration, distinguished from said measurement configuration by a difference affecting the measured value of said characterizing parameter, said apparatus (300) being **characterized in that** the characterizing parameter is a bone texture parameter, and **in that** it comprises means (302-308) comprising at least one calculation means and arranged and configured for:

- determining a fractal dimension of said greyscale image,
- correcting said fractal dimension with reference to said reference configuration, said correction providing a value, called corrected value, of said fractal dimension, and
- determining a value, called corrected value, of said characterizing parameter from said corrected value of said fractal dimension.

8. Correction/calibration apparatus (300) according to the preceding claim, in which the bone texture parameter is the Hurst parameter.

9. System (400) for determining a parameter for characterizing bone tissue, said system (400) comprising:

- imaging means (402, 404) for acquiring a greyscale image of said bone tissue, and
- a correction/calibration apparatus (300) according to one of claims 7 and 8.

10. System (400) according to the preceding claim, **characterized in that** the imaging means (402, 404) comprise a digital or analogue detector (404).

11. Use of the system according to any one of claims 9 and 10 for determining the bone texture of bone tissue.

| Détermination, par analyse de l'image en niveaux de gris d'une valeur mesurée d'un paramètre de caractérisation | 102 |

| Détermination, d'une valeur mesurée de la dimension fractale de l'image en niveaux de gris | 104 |

| Correction/calibration de la valeur mesurée de la dimension fractale par rapport à la configuration de référence | 106 |

| Correction/calibration de la valeur mesurée du paramètre de caractérisation en fonction de la valeur corrigée/calibrée de la dimension fractale | 108 |

100

**FIG. 1**

Acquisition d'une image en niveaux de gris
dans une configuration de mesure ⎤⟋202

Définition d'une région d'intérêt par
des repères anatomiques ⟋204

Acquisition d'une nouvelle image en
niveaux de gris ⟋206

100

200

**FIG. 2**

302    304    306    308

300

**FIG. 3**

402

300

404

400

**FIG. 4**

| PHANTOM | Configuration de mesure : $H_1$ HAMAMATSU mesurée | Configuration de référence : $H_1$ DEXELA mesurée | $H_{cor}$ HAMAMATSU corrigée selon l'état de la technique |
|---|---|---|---|
| 5161 | 0,598707941 | 0,435751089 | 0,45388049 |
| 5162 | 0,57517474 | 0,408175463 | 0,43603997 |
| 5169 | 0,707471552 | 0,559369919 | 0,536334184 |
| 5171 | 0,658541959 | 0,515225364 | 0,499240659 |
| MEAN | 0,634974048 | 0,479630459 | 0,481373826 |
| SD | 0,059728615 | 0,069896577 | 0,045280263 |

502

| PHANTOM | Configuration de mesure : $H_1$ HAMAMATSU mesurée | Configuration de référence : $H_1$ DEXELA mesurée | $H_{cor}$ HAMAMATSU corrigée selon l'invention |
|---|---|---|---|
| 5161 | 0,598707941 | 0,435751089 | 0,439100775 |
| 5162 | 0,57517474 | 0,408175463 | 0,412887143 |
| 5169 | 0,707471552 | 0,559369919 | 0,560252562 |
| 5171 | 0,658541959 | 0,515225364 | 0,505749888 |
| MEAN | 0,634974048 | 0,479630459 | 0,479497592 |
| SD | 0,059728615 | 0,069896577 | 0,066531704 |

504

## FIG. 5

602

604

**FIG. 6**

## RÉFÉRENCES CITÉES DANS LA DESCRIPTION

**Documents brevets cités dans la description**

- US 6377653 B

**Littérature non-brevet citée dans la description**

- Multifractal Radiographie Analysis Of Osteoporosis. **CALIGIURI P et al.** MEDICAL PHYSICS. AIP, 01 Avril 1994, vol. 21, 503-508